**Europäisches Patentamt**

**European Patent Office**

**Office européen des brevets**

(19)

(11) Veröffentlichungsnummer: **0 111 115**
**B1**

(12)

# EUROPÄISCHE PATENTSCHRIFT

(45) Veröffentlichungstag der Patentschrift:
18.03.87

(51) Int. Cl.⁴: **C 07 C 31/32**, B 01 J 8/02

(21) Anmeldenummer: **83110446.8**

(22) Anmeldetag: **20.10.83**

(54) **Verfahren und Vorrichtung zur kontinuierlichen Herstellung von Aluminiumalkoholaten.**

(30) Priorität: **04.12.82 DE 3244972**

(43) Veröffentlichungstag der Anmeldung:
**20.06.84 Patentblatt 84/25**

(45) Bekanntmachung des Hinweises auf die Patenterteilung:
**18.03.87 Patentblatt 87/12**

(84) Benannte Vertragsstaaten:
**BE FR GB NL**

(56) Entgegenhaltungen:
**EP-A-0 018 037**
**DE-B-1 251 297**
**DE-C-930 087**
**US-A-2 666 076**
**US-A-2 845 447**

(73) Patentinhaber: **Condea Chemie GmbH, Fritz-Staiger Strasse 15, D-2212 Brunsbüttel 1 (DE)**

(72) Erfinder: **Albert, Gert, Gustav- Frenssen- Strasse 26, D-2212 Brunsbüttel (DE)**
Erfinder: **Kamps, Manfred, Alten Hafen 21, D-2212 Brunsbüttel (DE)**
Erfinder: **Noweck, Klaus, Berliner Strasse 22, D-2212 Brunsbüttel (DE)**
Erfinder: **Reichenauer, Ansgar, Feldstrasse 55, D-2222 Marne (DE)**
Erfinder: **Scherf, Erich, Dr., Föhrer Strasse 11, D-2212 Brunsbüttel (DE)**
Erfinder: **Ziegler, Udo, Kurt- Schumacher- Ring 3, D-2212 Brunsbüttel (DE)**

(74) Vertreter: **UEXKÜLL & STOLBERG Patentanwälte, Beselerstrasse 4, D-2000 Hamburg 52 (DE)**

## Beschreibung

Die Erfindung betrifft ein kontinuierliches Verfahren zur Herstellung von Aluminiumalkoholaten durch Umsetzung von Aluminium in zerkleinerter Form mit einem Überschuß eines aliphatischen $C_3$-bis $C_{10}$-Alkohols, bei dem das von oben in einen Reaktor zugeführte Aluminium oberhalb eines Sieb- bzw. Gitterbodens im Reaktor mit einem Aluminiumalkoholat/Alkohol-Gemisch in Kontakt gebracht wird.

Bei der Umsetzung von Aluminium mit wasserfreiem Alkohol zur Herstellung von Metallalkoholaten ergeben sich wegen der bei dieser Umsetzung auftretenden hohen Wärmetönung erhebliche Schwierigkeiten sowohl beim Umsatz größerer Mengen als auch bei kontinuierlicher Durchführung des Verfahrens. Derartige Störungen werden beispielsweise durch ungleiche Aluminiumdosierung hervorgerufen oder dadurch, daß ein Teil des Aluminiums erst nach einiger Zeit unter momentaner Freisetzung größerer Wärmemengen reagiert.

Zur Behebung dieser Nachteile hat man gemäß US-PS 2 845 447 vorgeschlagen, die Umsetzung nicht mit einem Überschuß an Alkohol sondern mit einem solchen an Aluminium in flüssiger Phase durchzuführen, wobei das im Reaktor befindliche Aluminium mit einem im unteren Bereich des Reaktors vorhandenen Gemisch aus Aluminiumalkoholat und Alkohol in Kontakt gebracht wird, und das entstandene Aluminiumalkoholat mit dem noch nicht umgesetzten Alkohol zum Teil im Kreislauf dem Reaktor wieder zugeführt und zum anderen Teil isoliert wird. Bei diesem Verfahren mit überschüssigem Aluminium können durch Druckschwankungen oder aus anderen Gründen plötzlich größere Mengen des Gemisches aus Alkohol und Aluminiumalkoholat mit dem Aluminium in Kontakt kommen und dadurch größere Wärmemengen freisetzen, was zu einer Überlastung der Anlage führen kann. Darüber hinaus ergibt das Arbeiten mit unterstöchiometrischen Mengen an Alkohol geringere Reaktionsgeschwindigkeiten, die die Ausbeute verringern.

Ähnliche Nachteile hinsichtlich der Steuerung der Reaktion treten bei einem Verfahren gemäß US-PS 2 666 076 auf, bei dem mit einem im Reaktor befindlichen Überschuß an Aluminiumalkoholat/Alkohol-Gemisch gearbeitet und Aluminiumgranulat von oben in den Reaktor kontinuierlich zugeführt wird.

Bei einem anderen jedoch diskontinuierlichen Verfahren gemäß DE-PS 930 087 befindet sich das Aluminium in zerkleinerter Form auf einem Gitterboden eines Reaktors und wird mit dem in den Reaktor geleiteten Alkohol in Kontakt gebracht. Bei einem Ausbrechen der Reaktion wird die flüssige Phase durch den entstehenden Wasserstoffüberdruck in ein Ausgleichsgefäß gedrückt, so daß die flüssige Phase von dem auf dem Gitterboden befindlichen Aluminium getrennt wird. Dieses Verfahren arbeitet mit einem Alkoholüberschuß und kann bei einem Rückschlagen der flüssigen Phase zu einer schwer steuerbaren übermäßigen Umsetzung führen.

Die Erfindung hat sich die Aufgabe gestellt, ein Verfahren zur Herstellung von Aluminiumalkoholaten vorzuschlagen, das kontinuierlich und mit guten Ausbeuten störungsfrei durchführbar ist, wobei im Falle von etwa auftretenden Störungen die flüssige Phase sofort von dem Aluminium getrennt werden kann. Ferner kann bei dem erfindungsgemäßen Verfahren im Gegensatz zu den oben erwähnten oder zu anderen üblichen Verfahren einmal handelsübliches Aluminium anstelle von durch Zusatz von Metallen oder Metallverbindungen aktiviertem Aluminium und zum anderen ausschließlich reiner Alkohol, d.h. der zur Alkoholatbildung gewünschte Alkohol, verwendet werden, ohne daß, wie vielfach vorgeschlagen, noch schwerere Kohlenwasserstoffe in der flüssigen Phase zur Steuerung der Reaktion eingesetzt werden müssen, was die Reinheit des zu isolierenden Produktes gewährleistet.

Zur Lösung dieser Aufgabe wird daher ausgehend von einem Verfahren der eingangs gekennzeichneten Art vorgeschlagen, daß man das Aluminium auf ein im Bereich eines Sieb- bzw. Gitterbodens erzeugtes Sprudelbett aus einer Mischung aus Aluminiumalkoholat und Alkohol aufgibt, wobei die in den Reaktorsumpf ab- oder überlaufende Mischung aus Aluminiumalkoholat und Alkohol zu einem Teil im Kreislauf dem Reaktor über das Sprudelbett wieder zugeführt und zum anderen Teil isoliert wird.

Dieses Verfahren ermöglicht vorteilhafterweise, daß in dem durch die Verdampfung von Alkohol und die Entwicklung von Wasserstoff erzeugten Sprudelbett eine gute Durchmischung der Reaktionsteilnehmer im Sprudelbett und damit eine sofortige Umsetzung ermöglicht wird. Ein weiterer wesentlicher Vorteil beruht darauf, daß man bei auftretenden Störungen durch Unterbrechung der Zufuhr des Aluminiums und des Aluminiumalkoholat/Alkohol-Gemisches das Sprudelbett sofort in sich zusammenfallen lassen und Aluminium von der flüssigen Reaktionsmischung trennen kann.

Vorzugsweise wird das Aluminium in einer Aufschlämmung aus Alkohol und feinteiligem Aluminium als Suspension in den Reaktor und auf das Sprudelbett geführt. Dieses ermöglicht einmal die Zufuhr der beiden Reaktionsteilnehmer in einer kalten also nicht reaktionsfähigen Suspension und zum anderen die gesteuerte Zufuhr dieser Suspension in die auf reaktionsfähiger Temperatur gehaltene Flüssigphase, so daß wegen des in überstöchiometrischer Menge vorliegenden Alkohols mit günstigen Reaktionsgeschwindigkeiten gearbeitet werden kann.

Diese Art der Dosierung besitzt außerdem den Vorteil, daß sie unabhängig von der Geometrie der Aluminiumpartikel funktionsfähig ist, so daß Späne, Nadeln oder Grieß als Einsatzmaterialien Verwendung finden können.

Ferner ist es zweckmäßig, wenn man einen Teil der abgezweigten Kreislaufmischung aus Aluminiumalkoholat und Alkohol von oben in den Reaktor führt, um die Reaktion von Aluminium mit dem Alkohol gleichmäßiger ablaufen zu lassen. Aus dem gleichen Grund ist es von Vorteil, wenn man die Aufschlämmung aus Alkohol und feinteiligem Aluminium über eine Düse mit zusätzlichem Alkohol und/oder mit einer Aluminiumalkoholat/Alkohol-Mischung als Suspension in den Reaktor führt. Die Zufuhr von Alkohol sichert auch im oberen Bereich des Sprudelbettes ein hinreichendes Angebot von Alkohol, wodurch höhere Umsetzungsraten ermöglicht werden. Um das Sprudelbett in seiner vollen Wirkung zu halten und um einen gleichmäßigen Reaktionsablauf zu gewährleisten, ist es von Vorteil, wenn man die Kreislauf-Mischung aus Aluminiumalkoholat und Alkohol sowohl von oben, als auch über das Sprudelbett dem Reaktor zuführt, wobei das Verhältnis der von oben zugeführten zu den über das Sprudelbett zugeführten Volumenteil 1:2 bis 1:4 beträgt.

Ferner ist es vorteilhaft, daß von der überlaufenden Mischung aus Aluminiumalkoholat und Alkohol 35 bis 90 Volumenteile dem Reaktor zugeführt und 1 Volumenteil als Produkt isoliert werden.

Die Erfindung betrifft ferner eine Vorrichtung zur Durchführung des Verfahrens, die aus einem Reaktor mit einem Gitterboden oder Siebboden, einer im oberen Bereich des Reaktors befindlichen Zufuhrleitung für die Reaktionsteilnehmer und einer im unteren Bereich des Reaktors befindlichen sich in eine Rückführleitung zum Reaktor und in eine Produktenleitung aufteilenden Austrageleitung besteht, die dadurch gekennzeichnet ist, daß unterhalb des Sieb- bzw. Gitterbodens eine Wanne mit Zufuhrleitungen für das Aluminiumalkoholat/Alkohol-Gemisch und mit Ablaufbohrungen vorgesehen ist, und daß der in der Wanne befindliche Sieb- bzw. Gitterboden durch eine umlaufende Wand mit Durchbrechungen korbartig ausgebildet ist.

Vorzugsweise sind die Ablaufbohrungen in der Wanne im Verhältnis zu den Zufuhrleitungen für das Aluminiumalkoholat/Alkohol-Gemisch so bemessen, daß größere Volumenanteile des Aluminiumalkoholat/Alkohol-Gemisches in die Wanne gelangen, als in der gleichen Zeit dieses Gemisch durch die Ablaufbohrungen ablaufen kann. Dieses bedeutet, daß auch bei teilweise ablaufender flüssiger Phase immer ein Sprudelbett durch die größere Menge des zugeführten Aluminiumalkoholats/Alkohol-Gemisches aufrechterhalten wird. Vorzugsweise sollen je 1 Teil des aus den Ablaufbohrungen ablaufenden Aluminiumalkoholat/Alkohol-Gemisches 3 bis 15 und insbesondere 6 bis 8 Volumenteile dieses Gemisches dem Sprudelbett zugeführt werden.

Das Aluminium wird bei dem erfindungsgemäßen Verfahren wie üblich in zerkleinerter Form z.B. in Form von Nadeln, Spänen oder Granulat eingesetzt, wobei es nicht erforderlich ist, das Aluminium durch Metallverbindungen zu aktivieren.

Als Alkohole können aliphatische $C_3$-bis $C_{10}$-Alkohole und insbesondere $C_5$-bis $C_8$-Alkohole vorzugsweise solche mit gradkettigem Alkylrest verwendet werden.

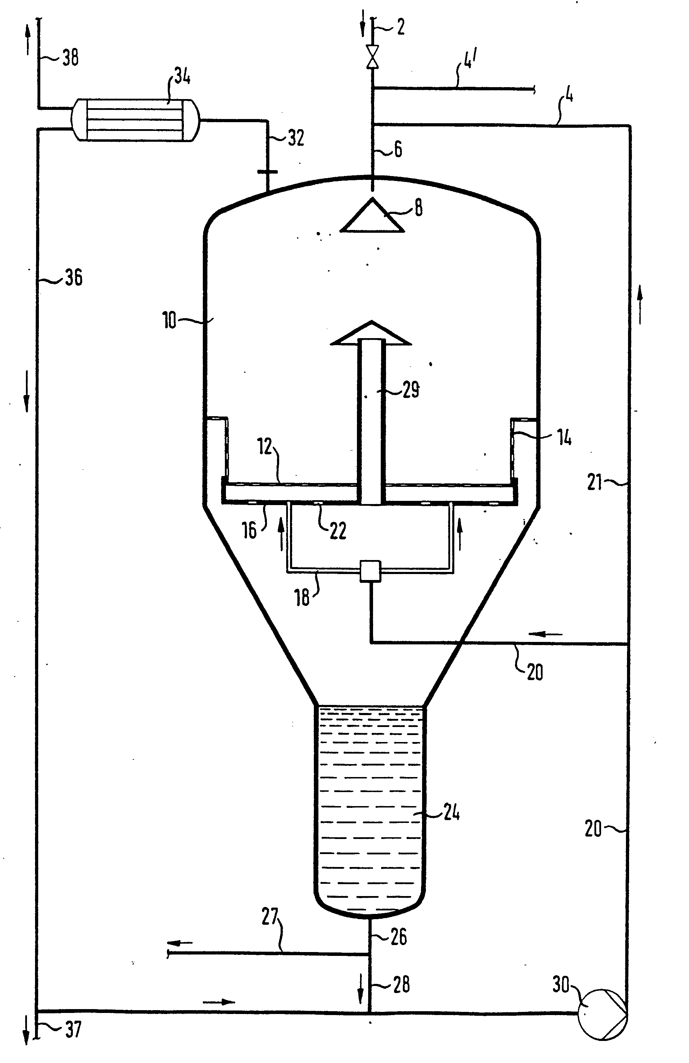

Im folgenden soll die Erfindung anhand der beiliegenden Zeichnung näher erläutert werden, die schematisch den erfindungsgemäßen Reaktor zeigt.

Über eine Leitung 2 wird aus einem hier nicht gezeigten Vorratsbehälter entweder feinteiliges Aluminium pneumatisch bzw. über eine Druckschleuse mechanisch zugeführt oder vorzugsweise als eine Suspension von feinteiligen Aluminium in dem zur Alkoholatbildung gewünschten Alkohol mittels einer Pumpe oder eines Flüssig - Flüssig - Ejektors aus dem gerührten Anmischbehälter über eine Zufuhrleitung 6 in den unter geringem Überdruck stehenden Reaktor 10 eingeführt. Der Feststoffgehalt der Aluminiumsuspension kann in einem Bereich von 5 bzw. 50 Gew.% Aluminium liegen. Weiterer Alkohol und/oder ein Aluminiumalkoholat/Alkohol-Gemisch können über getrennte Leitungen 4 bzw. 4' im Kopfbereich der Zufuhrleitung 6 zugeführt werden. Zur besseren Verteilung der Aluminiumsuspension sind unterhalb der Austrittsöffnung der Zufuhrleitung 6 Verteilerbleche 8 angeordnet.

Im Reaktor 10 ist ein Gitter- oder Siebboden 12 vorgesehen, der umlaufende Wände 14 mit entsprechenden Durchbrechungen besitzt und auf beliebige Weise im Reaktor befestigt ist. Unterhalb des Sieb- oder Gitterbodens befindet sich eine Wanne 16, die über mehrere Zufuhrleitungen 18 und über einen Verteilerkopf von einer Leitung 20 mit einer Mischung aus Aluminiumalkoholat und Alkohol versorgt wird. Das aus den Zufuhrleitungen austretende flüssige Gemisch gewährleistet im Bereich des Gitterbodens 12 ein Sprudelbett, in dem die Umsetzung zwischen dem Alkohol und dem Aluminium erfolgt.

Im Boden der Wanne 16 sind Ablauföffnungen 22 vorgesehen, deren Durchtrittsfläche gegenüber der der Zuleitungen 18 so bemessen ist, daß zwar ein Teil der flüssigen Phase aus der Wanne nach unten in den Reaktor abläuft, jedoch immer noch so viel Überschuß an flüssiger Phase in der Wanne vorhanden ist, daß die für das Sprudelbett erforderliche Flüssigkeitsmenge aufrechterhalten werden kann.

Das sich im Sprudelbett bildende Aluminiumalkoholat läuft mit dem vorhandenen Alkohol durch die Öffnungen im Wandbereich 14 des korbartigen Gitterbettes nach unten in den Reaktorsumpf ab. Das sich im unteren Bereich des Reaktors ansammelnde und an Aluminiumalkoholat angereicherte Gemisch aus Alkohol und Aluminiumalkoholat wird über eine Leitung 26 abgezogen und zum Teil über eine Leitung 28 und mittels einer Kreislaufpumpe 30 gegebenenfalls mit frischem Alkohol, wieder über die Leitung 20 in den Reaktor und zum anderen Teil über die Leitung 27 zur weiteren Aufarbeitung bzw. zur Isolierung des Aluminiumalkoholats geführt. Der bei der Reaktion entstehende Wasserstoff und der verdampfte Alkohol werden über eine Austrittsleitung 32 einem Kondensator 34 zugeführt, von dem der kondensierte Alkohol über eine Leitung 36 zur Auffrischung des aus dem Reaktorsumpf 24 abgezogenen und über die Leitungen 26 und 28 der Kreislaufpumpe 30 zugeführten Aluminiumalkoholat/Alkohol-Gemisches wieder in den Reaktor geleitet wird. Der Alkohol kann auch über eine weitere Leitung 37 abgeführt und z.B. mit der Aluminiumaufschlämmung oder direkt von oben in den Reaktor eingeleitet werden. Der im Kondensator anfallende und vom Alkohol befreite Wasserstoff wird über eine Leitung 38 abgezogen.

Die Leitung 20 steht über eine Abzweigleitung 21 mit der Zufuhrleitung 4 in Verbindung, so daß das Aluminiumalkoholat/Alkohol-Gemisch sowohl über das Sprudelbett als auch von Kopf in den Reaktor gegeben werden kann.

Zur Vermeidung von Druckunterschieden zwischen dem oberen und unteren Bereich des Reaktors ist ein vorzugsweise abgeschirmtes Druckausgleichsrohr 29 vorgesehen, welches den unteren Bereich des Autoklaven mit oberem Bereich verbindet.

Bei Auftreten von Unregelmäßigkeiten oder Störungen im Reaktionsablauf, die sich durch Druck- oder Temperaturschwankungen bemerkbar machen, kann durch ein sofortes Abstellen der Kreislaufpumpe für das Aluminiumalkoholat/ Alkoholgemisch und gleichzeitiges Unterbrechen der Zufuhr der Aluminium/Alkoholsuspension durch Schließen eines Ventiles in der Zufuhrleitung 6 eine Unterbrechung der Reaktion bewirkt werden. Beim Abstellen der Kreislaufpumpe fällt das Sprudelbett sofort in sich zusammen; die flüssige Phase läuft durch die Öffnungen 22 in den unteren Teil des Reaktors ab, wobei auf dem Siebboden nur eine geringe Menge an Aluminium aus der vorher zugeführten Aluminium/ Alkoholsuspension verbleibt. Mit dieser Anordnung wird bei einem Störfall innerhalb von 20 bis 120 Sekunden eine vollständige Trennung zwischen der festen und flüssigen Phase erreicht.

Bei dem erfindungsgemäßen Verfahren kann man das Sprudelbett mit der gewonnenen Aluminiumalkoholat/Alkoholmischung bzw. mit einer an frischem Alkohol angereicherten Mischung oder nur mit diesem Alkohol versorgen. Ebenso kan über die Zufuhrleitung 6 reiner Alkohol oder ein bereits Aluminiumalkoholat enthaltender Alkohol zugeführt werden. Wenn ein Teil des abgeweigten Kreislaufstromes mit der Aluminiumsuspension gemischt und von oben in den Reaktor geführt wird, erfolgt gleichzeitig eine Vorwärmung der zugeführten Frischmenge durch die aufsteigenden Alkoholdämpfe.

Beim Abfahren des Reaktors oder nachh einem Störfall entfällt ein Öffnen und Reinigen des Reaktors, da man mit der Kreislaufflüssigkeit und Alkohol den Reaktor solange betreiben kann, bis das im Siebkorb verbliebenen Aluminium abgebaut ist, worauf dann wieder frische Aluminium/Alkohol-Suspension zugegeben werden kann.

### Beispiel 1

In dem eingangs beschriebenen Reaktor aus Stahl mit einem Fassungsvermögen von 3 m$^3$ und einem Durchmesser im Siebbereich von 0,84 m wurden stündlich 110 kg Aluminiumnadeln mit einer Dicke von 0,4 bis 0,6 mm und einer Länge von etwa 4 bis 6 mm je m$^2$ Siebbodenfläche mit insgesamt 13,6 kg Hexanol je kg Aluminium zugeführt. Im Reaktor wurden je Stunde 0,83 m des aus Aluminiumalkoholat und Alkohol bestehenden Gemisches je kg Aluminium im Kreislauf zugeführt, und zwar sowohl durch die obere Zufuhrleitung 4 als auch durch die Zufuhrleitung 20 wobei das Verhältnis der zugeführten Mengen 1:3 betrug. Der Reaktordruck betrug 1,3 bar.

Unter diesen Reaktionsbedingungen bildete sich auf dem Siebboden eine Sprudelschicht von etwa 15 cm Höhe aus, deren Temperatur bei etwa 175° C lag. Die Ausbeute an Aluminiumalkoholat bezogen auf den Aluminiumeinsatz lag bei 99,4 %.

Bei einem simulierten Störfall wurden alle Dosiervorrichtungen und die Kreislaufpumpe abgeschaltet, worauf nach 30 Sekunden die gesamte Flüssigphase vom Siebboden in den unteren Bereich des Reaktors abgelaufen war.

### Beispiel 2

Es wurde analog Beispiel 1 gearbeitet, wobei jedoch jetzt die erforderliche Aluminiumalkoholat/Alkoholmischung nur über die Leitung 20 der Wanne 16 bzw. dem Sprudelbett zugeführt wurden. Die Ausbeute an Aluminiumalkoholat lag bezogen auf dem Aluminiumeinsatz bei etwa 99,1. Es konnten jedoch nur 100 kg Aluminium je m$^2$ Siebboden und Stunde umgesetzt werden.

**Beispiel 3**

In einen Versuchsreaktor, der in seinem Aufbau dem Reaktor gemäß Beispiel 1 entsprach, wurden über eine Dosiervorrichtung von oben Aluminiumgranulat zugeführt. Es wurden je Stunde 0,84 m$^3$ des aus Aluminiumalkoholat und Alkohol bestehenden Gemisches je kg eingesetzten Aluminiums im Kreislauf geführt, jedoch nur über die untere Zufuhrleitung (20).

Der Reaktorüberdruck betrug 0,3 bar. Die Temperatur in der Sprudelschicht lag bei etwa 175° C.

Bei einem Einsatz von 13,6 kg Hexanol je kg Aluminium und bei einer Ausbeute an Aluminiumalkoholat von 99,3 bezogen auf den Aluminiumeinsatz, konnten jedoch nur 98 kg Aluminiumgranulat je m$^2$ Siebboden und Stunde umgesetzt werden.

**Patentansprüche**

1. Verfahren zur kontinuierlichen Herstellung von Aluminiumalkoholaten durch Umsetzung von Aluminium in zerkleinerter Form mit einem Überschuß eines aliphatischen C$_3$-bis C$_{10}$-Alkohols, bei dem das von oben in einen Reaktor zugeführte Aluminium oberhalb eines Sieb- bzw. Gitterbodens im Reaktor mit einem Aluminiumalkoholat/Alkohol-Gemisch in Kontakt gebracht wird, dadurch gekennzeichnet, daß man das Aluminium von oben auf ein im Bereich des Sieb- bzw. Gitterbodens erzeugtes Sprudelbett aus einer Mischung aus Aluminiumalkoholat und Alkohol aufgibt, wobei die in den Reaktorsumpf ab- oder überlaufende Mischung aus Aluminiumalkoholat und Alkohol zu einem Teil im Kreislauf dem Reaktor über das Sprudelbett wieder zugeführt und zum anderen Teil isoliert wird.

2. Verfahren nach Anspruch 1, dadurch gekennzeichnet, daß man das Aluminium in einer Aufschlämmung aus Alkohol und feinteiligem Aluminium als Suspension in den Reaktor und auf das Sprudelbett führt.

3. Verfahren nach Anspruch 1 und 2, dadurch gekennzeichnet, daß man die Aufschlämmung aus Alkohol und feinteiligem Aluminium über eine Düse mit zusätzlichem Alkohol und/oder mit einer Aluminiumalkoholat/Alkohol-Mischung als Suspension in den Reaktor führt.

4. Verfahren nach Anspruch 1 bis 3, dadurch gekennzeichnet, daß man einen Teil der abgezweigten Kreislaufmischung aus Aluminiumalkoholat und Alkohol von oben in den Reaktor führt.

5. Verfahren nach Anspruch 1 bis 4, dadurch gekennzeichnet, daß man die Kreislauf-Mischung aus Aluminiumalkoholat und Alkohol sowohl von oben als auch über das Sprudelbett dem Reaktor zuführt, wobei das Verhältnis der von oben zugeführten zu den über das Sprudelbett zugeführten Volumenteile 1:2 bis 4 beträgt.

6. Verfahren nach Anspruch 1 bis 5, dadurch gekennzeichnet, daß von der in den Reaktorsumpf abgelaufenen Mischung aus Aluminiumalkoholat und Alkohol je 35 bis 90 Vol.Teile des dem Reaktor wieder zugeführten Gemisches 1 Vol.Teil als Produkt isoliert wird.

7. Vorrichtung zur Durchführung des Verfahrens nach Anspruch 1 bis 6 bestehend aus einem Reaktor mit einem Sieb- bzw. Gitterboden, einer im oberen Bereich des Reaktors befindlichen Zufuhrleitung für die Reaktionsteilnehmer und einer im unteren Bereich des Reaktors befindlichen sich in eine Rückfuhrleitung zum Reaktor und in eine Produktenleitung aufteilende Austrageleitung, dadurch gekennzeichnet, daß unterhalb des Sieb- bzw. Gitterbodens (12) eine Wanne (16) mit Zufuhrleitungen (18) für das Aluminiumalkoholat/Alkohol-Gemisch und mit Ablaufbohrungen (22) vorgesehen ist, und daß der in der Wanne befindliche Sieb- bzw. Gitterboden (12) durch eine umlaufende Wand (14) mit Durchbrechungen korbartig ausgebildet ist.

8. Vorrichtung nach Anspruch 7, dadurch gekennzeichnet, daß die Ablaufbohrungen in der Wanne im Verhältnis zu den Zufuhrleitungen für das Aluminiumalkoholat/ Alkohol-Gemisch so bemessen sind, daß je 1 Teil des aus den Ablaufbohrungen (22) ablaufenden Aluminiumalkoholat/Alkohol-Gemisches 3 bis 15 und vorzugsweise 6 bis 8 Volumenteile dem Sprudelbett zugeführt werden.

**Claims**

1. A process for the continuous production of aluminium alcoholates by reacting aluminium in comminuted form with an excess of an aliphatic C$_3$- to C$_{10}$- alcohol in which the aluminium being supplied to the reactor from above is brought into contact with the aluminium alcoholate/alcohol mixture above a perforated plate or grid tray within the reactor, characterized in that the aluminium is fed from above to a bubble bed made of a mixture of aluminium alcoholate and alcohol in the area of the perforated plate or grid tray, whereby one part of the mixture of aluminium alcoholate and alcohol flowing into the reactor sump or overflowing is recycled to the reactor over the bubble bed and the other part is isolated.

2. A process according to claim 1, characterized by adding the aluminium to the reactor and onto the bubble bed as a suspension of alcohol and finely divided aluminium.

3. A process according to claims 1 and 2, characterized by adding the suspension of finely divided aluminium and alcohol via a nozzle together with additional alcohol and/or an aluminium alcoholate/alcohol mixture as a suspension.

4. A process according to claims 1 to 3, characterized by supplying a portion of the branched off recycling mixture of aluminium alcoholate and alcohol to the reactor from above.

5. A process according to claims 1 to 4, characterized by supplying the recycling mixture of aluminium alcoholate and alcohol to the reactor both from above and also via the bubble bed, whereby the ratio of the portion supplied from above to the portion supplied via the bubble bed is 1:2 to 1:4 parts by volume.

6. A process according to claims 1 to 5, characterized by isolating from the mixture of aluminium alcohlate and alcohol overflowing into the sump of the reactor 1 part by volume as product for each 35 to 90 parts by volume of the mixture recycled to the reactor.

7. An apparatus suitable for carrying out the process according to claims 1 to 6 comprising a reactor having a perforated plate or grid tray, a supply line in the upper area of the reactor for the reactants and a charge line in the lower area of the reactor being subdivided into a return line to the reactor and a product line characterized in that below the perforated plate or grid tray (12) there being provided a tray (16) having supply lines (18) for the aluminium alcoholate/alcohol mixture and having drain holes (22), and that the perforated plate or grid tray (12) being situated in the tray is formed like a basket by a circumferential wall (14) with perforations.

8. An apparatus according to claim 7, characterized in that the drain holes in the tray in relation to the supply lines for the aluminium alcoholate/alcohol mixture are so dimensioned that for each 1 part of the overflowing aluminium alcoholate/alcohol mixture flowing out through the drain holes (22) 3 - 15 and preferably 6 - 8 parts by volume of the mixture are added to the bubble bed.

**Revendications**

1.- Procédé de préparation en continu d'alcoolate d'aluminium par réaction d'aluminium sous forme de fines particules avec un excès d'alcool aliphatique en $C_3$ à $C_{10}$, dans lequel l'aluminium introduit par le haut du réacteur au-dessus d'un plateau perforé ou grillagé est mis en contact dans le réacteur avec un mélange alcoolate d'aluminium/alcool, caractérisé en ce que l'on introduit l'aluminium par le haut sur un lit fluidisé à l'aide d'un mélange d'alcoolate d'aluminium et d'alcool, ce lit fluidisé étant créé dans le plateau perforé ou grillagé, le mélange d'alcoolate d'aluminium et d'alcool tombant dans le fond du réacteur ou y parvenant par un trop-plein étant en partie recyclé dans le réacteur par le lit fluidisé et en partie séparé.

2.- Procédé selon la revendication 1, caractérisé en ce que l'on introduit l'aluminium dans le réacteur et dans le lit fluidisé sous la forme d'une bouillie constituée d'alcool et de fines particules d'aluminium en suspension.

3.- Procédé selon la revendication 1 ou 2, caractérisé en ce que l'on introduit la bouillie d'alcool et d'aluminium finement divisé en suspension dans le réacteur par un ajutage en même temps que de l'alcool supplémentaire et/ou un mélange alcoolate d'aluminium/alcool.

4.- Procédé selon la revendication 1 à 3, caractérisé en ce que l'on introduit une partie du mélange recyclé dérivé d'alcoolate d'aluminium et d'alcool dans le haut du réacteur.

5.- Procédé selon la revendication 1 à 4, caractérisé en ce que l'on introduit dans le réacteur le mélange recyclé d'alcoolate d'aluminium et d'alcool aussi bien par le haut du réacteur que par le lit fluidisé, le rapport des volumes introduits par le haut à celui introduit par le lit fluidisé étant compris entre 1/2 et 4.

6.- Procédé selon la revendication 1 à 5, caractérisé en ce que du mélange d'alcoolate d'aluminium et d'alcool descendant dans le fond du réacteur, on sépare une partie en volume sous la forme de produit pour 35 à 90% en volume de mélange recyclé dans le réacteur.

7.- Dispositif de mise en oeuvre du procédé selon la revendication 1 à 6, constitué d'un réacteur comportant un fond perforé ou grillagé, une conduite d'introduction des participants à la réaction se trouvant dans le haut du réacteur, et d'une conduite d'évacuation disposée dans le bas du réacteur et sur laquelle se trouve une conduite de recyclage vers le réacteur et une conduite de prélèvement du produit, caractérisé en ce que, en-dessous du plateau perforé ou du plateau en grillage (12), se trouve un bassin (16) pourvu de conduites d'introduction (18) du mélange alcoolate d'aluminium/alcool et d'orifices d'évacuation (22) et en ce que, le plateau perforé ou le plateau en grillage (12) se trouvant dans le bassin est entouré d'une paroi (14) en forme de panier comportant des orifices de sortie.

8.- Dispositif selon la revendication 7, caractérisé en ce que les orifices d'écoulement (22) pratiqués dans le bassin ont des dimensions telles par rapport aux conduites d'introduction du mélange alcoolate d'aluminium/alcool que, pour une partie du mélange alcoolate d'aluminium/alcool s'écoulant par les orifices d'évacuation (22), de 3 à 15 et de préférence de 6 à 8 parties en volume sont recyclées dans le lit fluidisé.